(19) Europäisches Patentamt
European Patent Office
Office européen des brevets

(11) Publication number: **0 214 642 A2**

(12) **EUROPEAN PATENT APPLICATION**

(21) Application number: 86112407.1

(51) Int. Cl.⁴: **C07H 5/06**, A61K 31/70

(22) Date of filing: 08.09.86

(30) Priority: **09.09.85 DE 3532081**

(43) Date of publication of application: **18.03.87 Bulletin 87/12**

(84) Designated Contracting States: **BE CH DE FR GB IT LI LU NL SE**

(71) Applicant: **ORION-YHTYMÄ OY FERMION**
**P.O. Box 28**
**SF-02101 Espoo(FI)**

(72) Inventor: **Noujua, Ari Pekka**
**Kaunispääntie 3 A 1**
**SF-00970 Helsinki(FI)**
Inventor: **Rinne, Kari Yrjö**
**Itätuulenkuja 7 C**
**SF-02100 Espoo(FI)**
Inventor: **Tulisalo, Jukka Matti**
**Laajaniityntie 12 G 93**
**SF-01620 Vantaa(FI)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

(54) **Mixed salts of glucosamine sulphate and a process for the preparation of the same.**

(57) The present invention relates to mixed salts of glucosamine sulphate and to a process for the preparation of the same. The compounds according to the invention consist of a mixed salt of glucosamine sulphate and alkaline or earth alkaline metal halides e.g. potassium chloride corresponding to the formula:

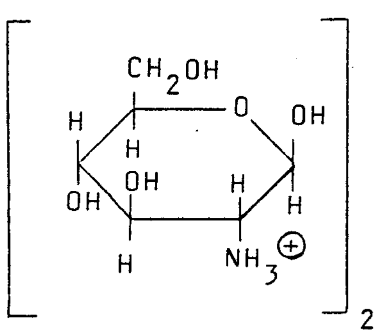

$.2K^{+}.SO_4^{=}.2Cl^{-}$

EP 0 214 642 A2

# MIXED SALTS OF GLUCOSAMINE SULPHATE AND A PROCESS FOR THE PREPARATION OF THE SAME

This invention relates to mixed salts of glucosamine sulphate and certain alkaline or earth alkaline metal halides such as, for example, potassium chloride, and to a process for the preparation of the same.

Glucosamine sulphate is a well-known substance in the treatment of, for example, rheumatic fever, pains resulting from arthrosis or arthritis and osteo-articular diseases.

The synthesis of glucosamine sulphate was described by Breuer in 1898 (Chem. Ber. 31, 2197) and, industrially, in GB-A-1 056 331, US-A-3 683 076 and CH-A-525 861.

The use of glucosamine sulphate in the preparation of pharmaceutical formulations is difficult owing to its instability since it is readily oxidized and strongly hygroscopic. Oral forms, thus, often contain antioxidants such as, for example, sodium hyposulphite to prevent oxidation of the amino group. According to GB-A-2 101 585 the hygroscopic nature of glucosamine sulphate can be considerably reduced by forming a mixed salt with sodium chloride.

It has now been found that glucosamine sulphate also forms mixed crystalline salts with potassium chloride, magnesium chloride, sodium bromide and potassium bromide. Iodides and all the lithium halides are too soluble in organic water-miscible solvents and calcium halides are too reactive to form homogeneous crystalline mixed salts with glucosamine sulphate in the present reaction conditions. In particular, the mixed salt of glucosamine sulphate and potassium chloride is very stable at ambient temperature and humidity.

The mixed salts according to the invention correspond to the formulae (I), (II) or (III):

(I) 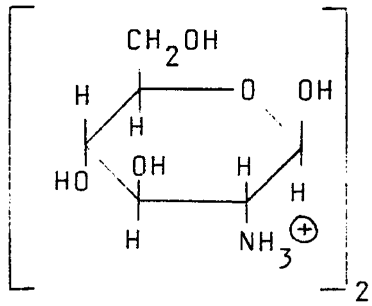 $.2K^{+}.SO_4^{=}.2X^{-}$ (X = Cl or Br)

(II) 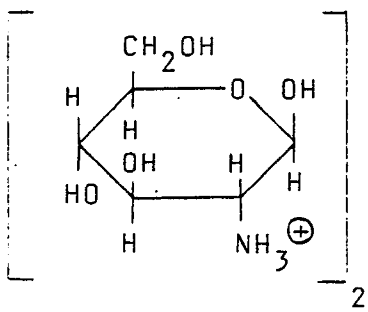 $.Mg^{2+}.SO_4^{=}.2Cl^{-}$

(III) 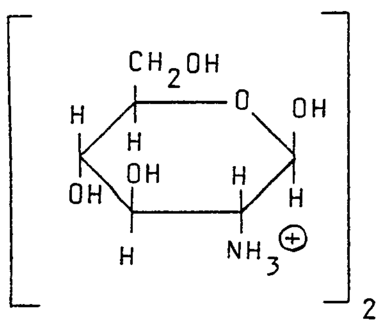 $.2Na^{+}.SO_4^{=}.2Br^{-}$

Glucosamine sulphate has been used as a sulphate itself or as a mixed salt of sulphate and sodium chloride. Glucosamine sulphate has several unfavourable properties, e.g. it has a highly hygroscopic nature and its amino group oxidises easily. The mixed salt of glucosamine sulphate and sodium chloride is stable, but its disadvantage lies in the use of sodium chloride.

The normal daily diet of an adult contains sufficient sodium chloride (1 to 5 g per day) to maintain the osmotic pressure of the blood and tissues. Often the daily intake with a normal diet is much higher though which is unfavourable for patients with hypertonus arterialis. The result is an increase in extracellular fluid and, to prevent this phenomenon from occurring, it is recommended that a part of the sodium chloride contained in the normal daily diet be replaced with other salts such as, for example, potassium chloride in table salt. Dosage ranges for the treatment with glucosamine sulphate are wide and it is possible that, during therapy, daily levels of 0,4 g of sodium chloride, which is from 8 to 40% of the normal daily diet, are reached.

A reported disadvantage with mixed salts of glucosamine sulphate and sodium chloride is an accummulation of sodium and water in the tissue, which is detrimental to patients suffering from cardiovascular diseases.

In rheumatic diseases, corticosteroids are often also administered during the glucosamine treatment and it is known that the treatment with corticosteroids often results in a lack of potassium which then has to be compensated with potassium chloride tablets.

It has now been found that it is possible to simplify the production of mixed salts of glucosamine sulphate and alkali or earth alkali metal halide compared with previous patents (such as GB-A-2 101 585). In this process, the glucosamine

base is converted to glucosamine sulphate and this intermediate is then converted without isolation to form a mixed salt of glucosamine sulphate and alkali or earth alkali metal halide.

The mixed salts are produced by dissolving the glucosamine base in water, adding a stoichiometric quantity of concentrated sulphuric acid to arrive at glucosamine sulphate and dissolving the stoichiometric quantity of alkaline or earth alkaline metal halide in the solution thus obtained. Precipitation is caused by adding a water-miscible organic solvent such as, for example, isopropanol. After addition of the liquid precipitant, the mixture is stirred at room temperature for about 14 hours to complete precipitation, cooled to 0°C and filtered.

The free glucosamine may be prepared according to Westphal and Holzmann (Chem. Ber. 75, 1274) by treating glucosamine hydrochloride with an ethanolic solution of a tertiary base such as triethylamine.

Example 1

Mixed salt of glucosamine sulphate and potassium chloride.

12,7 g (0,071 mol) of glucosamine was dissolved in 40 ml of water, cooled to 0°C and the mixture was adjusted to pH 4-5 by the slow addition of 3,5 g of 98 % $H_2SO_4$. The temperature was then raised to 20°C and 5,3 g (0,071 mol) of potassium chloride was added. Once the salt had dissolved the precipitation was carried out by using 240 ml of isopropanol, which was added over a period of 3 hours. To complete the precipitation the mixture was stirred for 14 hours at 20°C. After that the temperature was lowered to 0°C, the precipitate was isolated by filtration and dried at 50°C in reduced pressure. The yield was 19,7 g - (91,5 % of the theoretical) of $2(C_6H_{14}NO_5)^+.2K^+.SO_4^=.2Cl^-$, mp 210°C (dec.).

Example 2

Mixed salt of glucosamine sulphate and magnesium chloride.

The same procedure as that described in Example 1 was employed, using of 7,2 g (0,035 mol) of $MgCl_2.6H_2O$ instead of KCl. The yield was 17,9 g (92,9 %) of $2(C_6H_{14}NO_5)^+.Mg^{2+}.SO_4^=.2Cl^-$, mp 210 °C (dec.)

Example 3.

Mixed salt of glucosamine sulphate and sodium bromide.

The procedure was as described in Example 1 with the use of 7,3 g (0,071 mol) NaBr instead of KCl. The yield was 19,5 g (82,7 %) of $2(C_6H_{14}NO_5)^+.2Na^+.SO_4^=.2Br^-$, mp 200 °C (dec.)

Example 4

Mixed salt of glucosamine sulphate and potassium bromide.

The procedure was as described in Example 1 with the use of 8,4 g (0,071 mol) KBr instead of KCl. The yield was 20,7 g (84,9 %) of $2(C_6H_{14}NO_5)^+.2K^+.SO_4^=.2Br^-$, mp 200 °C (dec.).

Example 5

The procedure was as described in Example 1 with the use of ethanol instead of isopropanol. The yield was 18,8 g (87,5 %) of $2(C_6H_{14}NO_5)^+.2K^+.SO_4^=.2Cl^-$.

Example 6

The procedure was as described in Example 1 with the use of acetone instead of isopropanol. The yield was 20,3 g (94,5 %) of $2(C_6H_{14}NO_5)^+.2K^+.SO_4^=.2Cl^-$.

Stability test

To determine the stability of mixed salt of glucosamine sulphate and potassium chloride a sample was stored for 30 days at 20 °C and 75 % relative humidity. The results obtained are given in Table 1.

Table 1

Stability of mixed salt of glucosamine sulphate and potassium chloride at 20 °C and 75 % R.H.

| Time (days) | Assay |
|---|---|
| 0 | 99,4 % |
| 1 | 99,3 % |
| 2 | 98,7 % |
| 4 | 98,8 % |
| 8 | 98,7 % |
| 16 | 98;9 % |
| 30 | 98,7 % |

According to the results no remarkable degradation could be detected in mixed salt of glucosamine sulphate and potassium chloride during 30 days storage. On the other hand pure glucosamine sulphate decomposes completely at 20°C and 75 % R.H. in a few days.

**Claims**

1. A mixed salt of glucosamine sulphate and potassium chloride, magnesium chloride, sodium bromide or potassium bromide.

2. A mixed salt of glucosamine sulphate and potassium chloride.

3. A process for the preparation of a mixed salt of glucosamine sulphate and potassium chloride, magnesium chloride, sodium bromide or potassium bromide, characterized by first forming glucosamine sulphate from glucosamine and sulphuric acid and then forming the mixed salt by adding an approximately stoichiometric amount of the corresponding alkaline or earth alkaline metal halide and by precipitating the mixed salt by adding a water-soluble solvent.

4. A process in accordance with claim 3, characterized in that the formation of the mixed salt takes place at temperatures of from 0 to 40°C.

5. A process in accordance with claim 3 or 4, characterized in that the mixed salt is precipitated by adding isopropanol, mixing and cooling the mixture to a temperature of 0°C and filtering the precipitated product.

6. A process in accordance with any one of claims 3 to 5, characterized in that the precipitating solvent is ethanol, acetone or acetonitrile.